# EUROPÄISCHE PATENTSCHRIFT

(11) **EP 2 101 843 B1**
(45) Veröffentlichungstag und Bekanntmachung des Hinweises auf die Patenterteilung: **13.04.2016**
(21) Anmeldenummer: 08701055.9
(22) Anmeldetag: 10.01.2008
(51) Int. Cl.: A61M 1/36

(54) **VORRICHTUNG ZUR ENTFERNUNG VON LEUKOZYTEN AUS BLUT**
DEVICE FOR REMOVING LEUKOCYTES FROM BLOOD
DISPOSITIF D'ÉLIMINATION DE LEUCOCYTES DU SANG

(30) Priorität: 13.01.2007 DE 102007002059
(43) Veröffentlichungstag der Anmeldung: 23.09.2009
(73) Patentinhaber: 3M Innovative Properties Company, St. Paul, MN 55133-3427 (US)
(72) Erfinder: HEUSER, Frank, 42275 Wuppertal (DE); KÖNIG, Martin, 40225 Düsseldorf (DE); LEMKE, Horst-Dieter, 63785 Obernburg (DE); VON HARTEN, Bodo, 42119 Wuppertal (DE)
(74) Vertreter: Schröder, Richard
(86) Internationale Anmeldenummer: PCT/EP2008/000128
(87) Internationale Veröffentlichungsnummer: WO 2008/083965

(56) Entgegenhaltungen:
- EP-A- 0 285 812
- EP-A1- 1 570 897
- EP-A1- 2 113 297
- EP-A2- 0 732 142
- EP-B1- 0 093 677
- WO-A-2007/057065
- DD-A1- 233 946
- DE-A1- 2 300 312
- DE-A1-102004 063 504
- US-A- 3 730 959
- US-A- 4 367 139
- US-A- 4 750 918
- US-A1- 2013 247 760
- US-B2- 7 641 795
- US-B2- 8 318 022

## Beschreibung

Die Erfindung betrifft eine Vorrichtung zur Entfernung von Leukozyten aus Blut.

Blut besteht im wesentlichen aus Plasma und zellulären Elementen. Dazu zählen Erythrocyten (rote Blutkörperchen), Thrombozyten (Blutplättchen, Platelets) und Leukocyten (weiße Blutkörperchen). Zu den weißen Blutkörperchen zählen Lymphocyten, Monocyten und neutrophile Granulocyten (Neutrophile, PMN). Lymphocyten spielen eine entscheidende Rolle bei der spezifischen Immunität, Monocyten und neutrophile Granulozyten sind an der unspezifischen Immunabwehr oder Entzündungsreaktion beteiligte Zelltypen. Ihre Aufgabe ist es z.B., eingedrungene Mikroorganismen zu vernichten, die vorher von bestimmten körpereigenen Proteinen (etwa von C3b des Komplementsystems oder von Immunglobulin IgG) entsprechend als fremd markiert worden sind.

Haben die Zellen sich den eingedrungenen Mikroorganismen genähert, schütten sie sowohl Sauerstoffradikale als auch Proteasen aus, durch welche die Mikroorganismen abgetötet werden um danach phagozytiert zu werden. Wenn diese Reaktion nicht komplett abläuft oder außer Kontrolle gerät und chronisch wird, kann durch Freisetzung der agressiven Sauerstoffradikale und Proteasen auch körpereigenes Gewebe geschädigt werden. Während der Entzündung kommt es zwischen allen Zelltypen zu einer intensiven Kommunikation und Koordination, die sich u.a. verschiedener Cytokine bedient. Die Reaktion ist sehr komplex und noch nicht vollständig aufgeklärt. Sie führt aber letztlich zu den klinisch zu beobachtenden Symptomen der Entzündung von Schwellung, Rötung und Fieber. Charakteristisch ist u.a. ein Anstieg von im Knochenmark gebildeten und im Blut zirkulierenden Monocyten und neutrophilen Granulocyten und von bestimmten Botenstoffen. Neben den schon genannten proinflammatorischen Cytokinen werden auch die Komplementproteine C3a und C5a gebildet, die durch Aktivierung der Proteine C3 und C5 entstehen und die als Maß der Komplementaktivierung während der Entzündungs- oder Akutphasereaktion dienen.

Bei einer Reihe von entzündlichen Erkrankungen werden heute extrakorporale Therapien eingesetzt, etwa bei der Collitis ulcerosa, dem Morbus Crohn und der rheumatoiden Arthritis. Nach dem gegenwärtigen Stand der Technik werden dafür dem Patienten eine gewisse Anzahl von Zellen (wahrscheinlich speziell Monocyten und neutrophile Granulocyten) entfernt, indem das Blut des Patienten extrakorporal rezirkuliert und mit einem Zellfilter behandelt wird. Beispielsweise werden Säulen, die mit Partikeln, bzw. Beads aus Zelluloseacetat gefüllt sind, als Leucocytenfilter verwendet. Hier erfolgt die Entfernung hauptsächlich über Zelladsorption von Zellen an der Oberfläche der Beads. Solche Produkte sind bereits kommerziell erhältlich. Hier wird Blut durch eine Säule, die Zelluloseazetat Kugeln enthält, geleitet. Die Zelluloseazetat-Kugeln reduzieren speziell die im Blut enthaltenen Granulozyten und Monozyten durch Adsorption.

US 6,498,007 offenbart ein Verfahren zur Entfernung von Leukozyten aus Blut durch Adsorption an einen Träger. Dabei wird Blut mit diesem Träger in Kontakt gebracht, vorzugsweise in Form von sog. Beads, wobei der Träger eine höhere Affinität zu infizierten, aktivierten oder defekten Leukozyten aufweist als zu nicht infizierten, aktivierten bzw. defekten Leukozyten.

Alternativ werden auch Vliese oder Gewebe für die extrakorporale Entfernung von Leukozytenfilter eingesetzt. Beispielsweise werden für die Entfernung von Leukocyten aus einer Blutkonserve für die Transfusion (etwa eines Erythrocyten- oder eines Plateletkonzentrats) Produkte auf der Basis von Vliesen verwendet, bei denen die Zellentfernung überwiegend über mechanische Filtration mittels des Vlieses stattfindet. Für die Transfusion werden typischerweise Batches von 500 ml Blut in weniger als einer halben Stunde gefiltert. Der Prozeß läuft gravitationsbetrieben im single pass und nicht im Kreislauf ab. Damit ein Zellfilter im extrakorporalen Kreislauf eingesetzt werden kann, müssen etwa 1-6l Blut pumpengetrieben für einige Stunden gefiltert werden können. Dafür sind Polypropylenvliese in einem zylindrischen Gehäuse mit einem zuführenden Anschluss an der Stirnseite und einem abführenden Anschluss an der gegenüberliegenden Stirnseite kommerziell erhältlich. Mit dem dort verwendeten Vlies werden Leukozyten aufgrund von Filtrations- und Adsorptionseffekten zurückgehalten.

WO 95/18665 offenbart einen Filter und ein Verfahren zur Entfernung von Leukozyten und virusinaktivierenden Stoffen aus Plasma oder anderen Blutfraktionen. Der Filter basiert auf einem Netz aus textilen Fasern. An dem Netz sind Liganden mit hoher Affinität zu virusinaktivierenden Stoffen bzw. Leukozyten kovalent gebunden. Es handelt sich hierbei um eine selektive, aber technisch sehr aufwändige Methode, da die Liganden direkt oder über Linker an eine Polymermatrix zu binden sind.

Die Rückhaltung der Leukozyten mit solchen Filtern beruht auf einem Zelltrapping im Faservlies sowie einer mehr oder weniger starken Adsorption der Zellen an der Faseroberfläche. Allerdings werden bei diesen Verfahren die verschiedenen Blutzellen mechanisch sehr stark belastet, was zu einer Zellaktivierung oder gar zur Zerstörung der Blutzellen führen kann.

Ein wesentlicher Nachteil der bestehenden Vorrichtungen und Verfahren besteht darin, dass die verschiedenen Zelltypen nicht gezielt oder spezifisch adsorbiert werden können und dass neben den Monocyten und Granulozyten auch Lymphozyten, Thrombozyten und Erythrocyten adsorbiert werden. Dies kann, je nach Indikation, entweder gar nicht notwendig oder sogar schädlich für den Patienten sein. Einen Spezialfall stellt die Adsorption von Thrombozyten dar. Thrombozyten sind nach Aktivierung zentral an der Blutgerinnung beteiligt. Damit es während des extrakorporalen Kreislaufes nicht zur Blutgerinnung kommt, muß, etwa durch Gabe von Heparin als Antikoagulanz, medikamentös gegengesteuert werden. Kommt es trotz Antikoagulanz zur Blutgerinnung, führt das zu einem verstopften Filter.

Ein weiterer Nachteil von konventionellen Leukocytenfiltern besteht in der häufig schwierigen Handhabung vordem klinischen Einsatz, z.B. in Bezug auf Entlüftung. Luftblasen stellen in einem extrakorporalen Kreislauf potentiell eine Gefährdung des Patienten dar, sind also sehr unerwünscht. Je einfacher eine Entfernung des im Filters vorhandenen Luft möglich ist, desto besser die Handhabung und sicherer die Anwendung.

Aufgabe der vorliegenden Erfindung ist es daher, eine einfache und im Einsatz effiziente Vorrichtung zur Reduzierung von Leukozyten aus Blut zur Verfügung zu stellen, bei dem die Nachteile des Standes der Technik zumindest verringert sind.

Diese Aufgabe wird gelöst durch eine Vorrichtung zur Reduzierung von Leukozyten aus Blut umfassend eine Vielzahl von Hohlfäden auf Basis organischer Polymere, wobei die Hohlfäden ein Lumen und eine das Lumen umschließende Wand mit einer inneren, lumenseitigen Oberfläche und einer äußeren Oberfläche aufweisen, wobei die Hohlfäden in einem zylindrischen Gehäuse mit einer Einlasseinrichtung und einer Auslasseinrichtung vorliegen, wobei zwischen den Hohlfäden und dem Gehäuse ein Außenraum gebildet wird, der über die Einlasseinrichtung und die Auslasseinrichtung für ein Fluid zugänglich ist, und die Anordnung der Hohlfäden der Vorrichtung einen hohen Grad an Ordnung aufweist, wobei unter einem hohen Grad an Ordnung zu verstehen ist, dass ein hoher Anteil der Fäden entlang ihrer Erstreckungsrichtung nebeneinander angeordnet sind, dadurch gekennzeichnet, dass die Enden der Hohlfäden der Vorrichtung durch Einbettung in eine Vergussmasse, durch Schweißen oder Kleben verschlossen sind, dass nur die äußeren Oberflächen der Hohlfäden für ein Fluid zugänglich und die Lumina der Hohlfäden nicht für ein Fluid zugänglich sind und dass die Hohlfäden auf Basis organischer Polymere eine Bildung des Komplementaktivierungsproduktes C5a in einer Konzentration von mindestens 10 µg pro m² Fadenoberfläche bewirken.

Die Vielzahl von Hohlfäden auf Basis organischer Polymere befindet sich in einem Gehäuse mit einer Einlasseinrichtung und einer Auslasseinrichtung. Dabei sind die Hohlfäden so in das Gehäuse eingebracht, dass um die Fasern herum ein durch das Gehäuse begrenzter und von Blut durchströmbarer Außenraum ausgebildet wird, wobei gleichzeitig die Einlasseinrichtung und Auslasseinrichtung am Gehäuse so ausgeführt sind, dass nur der Außenraum um die Hohlfäden durchströmbar ist. Die inneren, lumenseitigen Oberflächen der Hohlfäden bzw. die Lumina der Hohlfäden sind bei der erfindungsgemäßen Vorrichtung nicht zugänglich, d.h. die inneren Oberflächen der Hohlfasern sind nicht an- bzw. durchströmbar. Das Gehäuse weist daher auch keine entsprechenden Ein- und/oder Auslasseinrichtung zur Anströmung der inneren Oberflächen auf.

Wesentlich für die Anwendung der erfindungsgemäßen Vorrichtung zur Entfernung von Leukozyten aus Blut ist, dass der Außenraum um die Hohlfasern von Blut durchströmbar ist, dass also die Hohlfasern an ihren äußeren Oberflächen umströmbar sind. Wie bereits in der noch nicht veröffentlichten internationalen Patentanmeldung PCT/EP2006/008585 dargelegt, bringt eine Umströmung der Hohlfäden an ihrer äußeren Oberfläche besondere Vorteile hinsichtlich der Adsorption von Leukozyten gegenüber einer Durchströmung der Lumina der Hohlfäden. Wahrscheinlich ist, bedingt durch die laminare Strömung in den Hohlfäden, der Stoffaustausch des Blutes mit der inneren Hohlfadenoberfläche im Vergleich zur Anströmung der äußeren Hohlfadenoberfläche stark verringert.

Eine Zugänglichkeit allein der äußeren Oberfläche der Hohlfäden erlaubt zum einen eine einfache Konstruktion der erfindungsgemäßen Vorrichtung. Zum anderen wird gleichzeitig vermieden, bedingt durch die Druckverhältnisse im Außenraum um die Hohlfäden, dass Blut oder Blutbestandteile in die Wand der Hohlfäden eindringen können und es dort zu Agglutinationsreaktionen kommt.

Die ausschließliche Zugänglichkeit der äußeren Oberfläche der Hohlfäden bzw. des Außenraumes um die Hohlfäden und damit die Nichtzugänglichkeit der Lumina der Hohlfäden wird erfindungsgemäß dadurch realisiert, dass die Enden der Hohlfäden verschlossen sind. Dazu können die Hohlfaserenden so in eine mit der Gehäuseinnenseite verbundenen Vergussmasse eingebettet werden, dass die Enden der Hohlfasern in der Vergussmasse liegen und durch die Vergussmasse verschlossen sind. Die Hohlfäden können dabei in einer bevorzugten Ausführungsform der erfindungsgemäßen Vorrichtung mit ihren Enden getrennt in Vergussmassen eingebettet sein und sich zwischen diesen Vergussmassen im wesentlichen geradlinig erstrecken. In diesem Fall liegt die Vielzahl von Hohlfäden als Fadenbündel mit im wesentlichen parallelen Hohlfäden vor. Die beidseitige Einbettung ist im Hinblick auf die Positionierung der Hohlfäden besonders vorteilhaft. Die Hohlfäden können aber auch mit beiden Enden in derselben Vergussmasse eingebettet sein, wobei die sich ausbildende U-förmige Schlaufe dann umströmbar ist. Die Hohlfäden können dabei in ihrer Gesamtheit eine Schlaufe bilden oder jede Hohlfaser für sich bildet eine Schlaufe und ist mit beiden Enden in derselben Vergussmasse eingebettet. Die Hohlfäden können auch nur mit einem Ende eingebettet sein und das andere, freie Ende ist dann z.B. durch Schweißen oder Kleben verschlossen und damit die innere Oberfläche nicht zugänglich.

Das Gehäuse kann über seine gesamte Länge einen konstanten Durchmesser aufweisen. Bevorzugt ist jedoch, dass das Gehäuse im Bereich Einlass bzw. Auslass einen erweiterten Durchmesser aufweist, der als Strömungsverteiler dient und für einen gleichmäßigen Blutvolumenstrom über den gesamten Strömungsquerschnitt sorgt.

Ein- und Auslasseinrichtung können in der erfindungsgemäßen Vorrichtung am Gehäusemantel angebracht sein, wobei sich die Einlasseinrichtung vorzugsweise an einem Ende des Gehäusemantels und die Auslasseinrichtung am anderen Ende des Gehäuses befindet. Ein- und Auslasseinrichtung können auf der gleichen Gehäuseseite vorliegen, vorteilhafter ist allerdings eine Anbringung auf gegenüberliegenden Seiten, bzw. eine um mindestens 90°, bevorzugt um 180° versetzte Anbringung.

Das Verschließen der Hohlfäden kann wie ausgeführt dadurch erfolgen, dass sie in eine Vergussmasse eingebettet, verschweißt oder geklebt werden. Unter die Erfindung fallen aber auch Ausführungsformen, bei denen die Hohlfadenenden nicht direkt verschlossen sind, sondern z.B. so in eine Vergussmasse eingebettet sind, dass die Enden nicht durch die Vergussmasse verschlossen sind und die Hohlfäden durch dichte Gehäusekappen verschlossen sind, wodurch die Lumina der Hohlfäden nicht für ein Fluid zugänglich sind.

Ein- und Auslasseinrichtung können in einer bevorzugten Ausführungsform auch an den Stirnseiten des Gehäuses vorliegen. Im Falle, dass die Hohlfäden an ihren Enden in eine Vergussmasse eingebettet sind, sind bei mittiger stirnseitiger Anbringung der Ein- bzw. Auslasseinrichtung die Vielzahl der Hohlfäden dann ringförmig mit ihren Enden in Vergussmasse eingebettet, wobei der Innendurchmesser der ringförmigen Anordnung der Hohlfäden mindestens so groß wie der Außendurchmesser der Ein- bzw. Auslasseinrichtung sein sollte. Die Ein- bzw. Auslasseinrichtung ist so ausgebildet, dass sie zentral von der Stirnseite durch die Vergussmasse in das Gehäuseinnere, d.h. in den Außenraum um die Hohlfäden geführt wird.

Natürlich können die verschiedenen Möglichkeiten des Verschließens der Hohlfäden und des Anbringens auch miteinander kombiniert werden. So kann beispielsweise eine stirnseitige Ein- oder Auslasseinrichtung an einem Ende des Gehäuses mit einer Ein- oder Auslasseinrichtung seitlich am anderen Ende des Gehäusemantels kombiniert sein. Auch ist es möglich, dass die Hohlfadenenden an der Einlassseite eingebettet und an der Auslassseite nur durch Kleben oder Schweißen verschlossen sind und ansonsten frei liegen. Die Gehäuseseite, an der das freie Ende der Hohlfäden liegt kann durch eine Endkappe verschlossen sein, wobei die Ein- bzw. die Auslasseinrichtung entweder am Gehäusemantel oder stirnseitig an der Endkappe angebracht oder durch die Vergussmasse geführt sein kann.

In einer weiteren bevorzugten Ausführungsform liegen die Hohlfäden in mehreren Lagen paralleler Hohlfäden vor, wobei die Lagen paralleler Hohlfäden besonders bevorzugt als Matten ausgeführt sind. Die Hohlfäden innerhalb jeder Matte werden von mehreren, durch Web- oder Wirkverfahren eingebrachten Querfäden, gehalten. Derartige Anordnungen paralleler Hohlfäden sind z.B. in EP 285 812 beschrieben. Die Hohlfadenmatte besteht in einer besonders bevorzugten Ausführungsform aus einem einzelnen mäanderförmig positionierten Hohlfaden der ebenfalls durch mehrere, durch Web- oder Wirkverfahren eingebrachte, Querfäden gehalten wird. In dieser Anordnung müssen für die ganze Matte lediglich die zwei Enden des Hohlfadens verschlossen werden, damit die gesamte innere Oberfläche nicht zugänglich ist.

Die Hohlfadenmatten können zum Einsatz in der erfindungsgemäßen Vorrichtung in mehreren Lagen, bevorzugt 10 - 200 Mattenlagen, übereinander gelegt werden, wobei jede Mattenlage bevorzugt 3 - 30 Hohlfäden pro cm enthält. Bevorzugt kreuzen sich die parallelen Hohlfäden einer Mattenlage mit den Hohlfäden der benachbarten Mattenlage in einem Winkel zwischen 10° und 90°, besonders bevorzugt in einem Winkel zwischen 10° und 40°. Die Hohlfäden sind mit ihren Enden bevorzugt getrennt in Vergussmassen eingebettet und durch diese Vergussmassen dicht verschlossen. Diese bevorzugte Ausführungsform der erfindungsgemäßen Vorrichtung zeichnet sich durch einen besonders niedrigen Druckverlust aus. Darüber hinaus sorgen die sich kreuzenden Mattenlagen im Betrieb der erfindungsgemäßen Vorrichtung für eine ausgezeichnete Strömungsverteilung und eine gleichmäßige Blutfilmdicke.

Die Hohlfadenmatten können zum Einsatz in der erfindungsgemäßen Vorrichtung auch als Wickelkörper ausgeführt sein und in einem zylindrischen Gehäuse mit kreisförmigem Querschnitt vorliegen. Die Hohlfäden können in so in Vergussmasse eingebettet sein, dass die Hohlfadenenden innerhalb der Vergussmasse liegen und durch diese verschlossen sind. In dieser Ausführungsform reicht es aber auch aus, wenn das Gehäuse mit Endkappen oder Vergussmasse verschlossen und die Hohlfadenmatte nur in das Gehäuse eingelegt ist, wobei die Enden der Hohlfasern, wenn diese nicht in der Vergussmasse eingebettet sind, verschlossen sein müssen, z.B. durch Kleben oder Verschweißen.

Um zu vermeiden, dass die Strömungskanäle zwischen den Hohlfasern zu eng bzw. zu klein werden und sich so Bereiche bilden, die nicht durchströmbar sind, beträgt in einer bevorzugten Ausführungsform der erfindungsgemäßen Vorrichtung der Außendurchmesser der Hohlfäden mindestens 150 µm, bevorzugt mindestens 250 µm. Derartige Hohlfäden lassen sich gleichzeitig einfach handhaben und zeichnen sich aufgrund ihrer Hohlstruktur trotz des geforderten Mindestdurchmesser durch geringes Gewicht und geringen Materialaufwand aus. Des weiteren ist es bevorzugt, dass der Außendurchmesser der Hohlfäden 2000 µm nicht überschreitet, da ansonsten eine zu geringe volumenbezogene Oberfläche zur Adsorption zur Verfügung steht.

Um sicher zu gewährleisten, dass alle Hohlfäden gleichermaßen mit Blut anströmbar sind, können die Hohlfäden in einer bevorzugten Ausführungsform der erfindungsgemäßen Vorrichtung auch zueinander beabstandet angeordnet werden, beispielsweise über sog. Abstandsfäden (Spaceryarn). Eine Beabstandung der Fäden ist im Hinblick auf die Vermeidung eines Siebeffektes besonders vorteilhaft. Solche Abstandsfäden sind besonders vorteilhaft, da dadurch ein gleichmäßiger Abstand zwischen im wesentlichen parallel vorliegenden Hohlfäden gewährleistet wird. Anordnungen mit solchen Abstandsfäden werden beispielsweise in der EP 732 141 oder der EP 285 812 beschrieben. Vorzugsweise bestehen die Abstandsfäden aus dem gleichen Material wie die Vielzahl paralleler Fäden. Es ist aber auch möglich, durch Verwendung spezieller Fadenmaterialien als Abstandsfaden, die Anzahl weiterer im Blut enthaltener Zelltypen zu reduzieren.

Um eine Schädigung der im Blut enthaltenen Zellen zu vermeiden, ist es wichtig, dass das Hohlfadenmaterial so beschaffen ist, dass Blut nicht in das Hohlfadenmaterial eindringen kann bzw. dass das Hohlfadenmaterial nicht von Blut durchströmbar ist. Dies wird zum einen erreicht durch den Verschluss der Hohlfäden. Zum anderen werden bevorzugt Hohlfäden mit einer dichten Oberfläche oder einer porösen Oberfläche mit einer maximalen Porenweite von 0,1 µm eingesetzt.

Als Hohlfäden sind Hohlfasermembranen mit dichter oder poröser Struktur bestens geeignet. Derartige Membranen werden beispielsweise zur Blutbehandlung bei der Dialyse eingesetzt. Die Porenweite der Hohlfasermembranen ist dabei so zu wählen, dass die Membranwand nicht von Blut durchströmbar ist, d.h. dass das Blut im wesentlichen nicht in den Hohlfaden, bzw. in die Hohlfasermembran eindringen kann.

Die Anzahl der Hohlfäden in der erfindungsgemäßen Vorrichtung liegt bevorzugt im Bereich von 2000 bis 20000 Hohlfäden, besonders bevorzugt im Bereich von 4000 bis 14000 Hohlfäden.

Der Füllgrad des Gehäuses mit den Hohlfäden aus organischen Polymeren sollte zwischen 10% und 70%, bevorzugt zwischen 30% und 60% betragen. Da die Fäden, abhängig vom Fadenmaterial, beim Kontakt mit Flüssigkeit unterschiedlich stark quellen können, ist die Bestimmung des Füllgrades des Gehäuses mit Fäden in gequollenem Zustand zu ermitteln. Bei stark quellenden Fäden, wie beispielsweise solche auf Zellulosebasis, sind deutliche Unterschiede bezüglich des Fadendurchmessers in gequollenem bzw. nicht gequollenem Zustand zu beobachten. Aufgrund der Quellung ergeben sich daher unterschiedliche Füllgrade, wenn die Fäden in trockenem Zustand vorliegen. Fäden, die nicht oder nahezu nicht quellen, wie beispielsweise solche aus Polysulfon zeigen dagegen keinen oder nur einen geringen Unterschied bei der Bestimmung des Füllgrades in gequollenem bzw. nicht gequollenem Zustand.

Der Füllgrad des Gehäuses ist in dem vorgegebenen Bereich zu limitieren, einerseits um eine hinreichend große Faseroberfläche zur Verfügung zu stellen, andererseits um einen Siebeffekt bei der Reduzierung von Leukozyten in dem erfindungsgemäßen Verfahren zu vermeiden.

Bevorzugt weist die erfindungsgemäße Vorrichtung ein Länge zu Durchmesser Verhältnis von mindestens 3:1, besonders bevorzugt von mindestens 5:1. Besonders gute Ergebnisse werden mit Vorrichtungen erzielt, die ein Länge zu Durchmesser Verhältnis von mindestens 10:1 aufweisen. So kann im Betrieb gewährleistet werden, dass die Kontaktzeit ausreichend lang und die Strömungsgeschwindigkeit des Blutes durch die erfindungsgemäße Vorrichtung nicht zu gering ist.

Unter einem hohen Grad an Ordnung im Sinne der vorliegenden Erfindung ist zu verstehen, dass die Fäden in ähnlicher Anordnung zueinander liegen bzw. dass ein hoher Anteil der Fäden entlang ihrer Erstreckungsrichtung nebeneinander angeordnet sind. Die Fäden liegen nicht als Vlies, Wirrfäden oder Wirrfasermatten vor, sondern die Anordnung der Fäden hat eine regelmäßige Struktur. Die Fäden weisen zwischen ihren Enden in ihrer Lage zueinander einen hohen Grad an Ordnung auf. Theoretisch hat ein Bündel gerader Fäden, die parallel zueinander liegen, den höchsten Grad an Ordnung. Einen hohen Grad an Ordnung im Sinne der vorliegenden Erfindung weist auch ein Bündel gewellter bzw. ondulierter Fäden auf, wobei die Fäden des Fadenbündels alle die gleiche Erstreckungsrichtung aufweisen. Auch ist unter einem hohen Grad an Ordnung im Sinne der vorliegenden Erfindung ein Faserbündel umfasst, das in einer Schlaufe gelegt ist. Auch hier ist die Anordnung der Fäden zueinander ähnlich. Ebenfalls ist unter einem hohen Grad an Ordnung zu verstehen, dass mindestens 30 % der Fäden parallel liegen. Des weiteren sind Fäden umfasst, die in mehreren Lagen vorliegen, wobei die Fäden innerhalb einer Lage im wesentlichen parallel zueinander angeordnet sind. Die parallelen Fäden einer Lage können sich allerdings mit den parallelen Fäden einer anderen Lage kreuzen. Derartige Anordnungen sind in der EP 285 812 beschrieben. Von den erfindungsgemäßen Anordnungen mit einem hohen Grad an Ordnung nicht umfasst sind Vliese oder Wirrfasermatten, in denen die Fasern völlig ungeordnet vorliegen und miteinander vermischt sind. Im Vergleich zu Vliesen weist die erfindungsgemäße Anordnung der Fäden mit einem hohen Grad an Ordnung eine höhere Oberfläche und bei Anwendung des erfindungsgemäßen Verfahrens eine gleichmäßige Blutfilmdicke auf. Der hohe Grad an Ordnung sorgt dafür, dass das an den Fäden vorbeiströmende Blut vergleichsweise geringe Turbulenzen aufweist und die im Blut enthaltenen Zellen einer geringen Scherbelastung ausgesetzt sind. Zudem sorgt der hohe Grad an Ordnung dafür, dass die Bildung von Toträumen und bevorzugte Kanälen, sog. Shunts, weitgehend vermieden wird. Hierdurch wird eine besonders schonende Blutbehandlung erreicht.

Der hohe Grad an Ordnung sorgt auch dafür, dass die Reduzierung der Leukozytenzahl im Wesentlichen nicht durch einen Siebeffekt hervorgerufen wird, wie dies z.B. bei einem Vlies der Fall wäre, sondern durch Adsorptionseffekte, wodurch eine besonders schonende Blutbehandlung ermöglicht wird. Zudem bewirkt der bei einem Vlies auftretende Siebeffekt auch zwangsläufig eine ungewollte Reduzierung anderer zellulärer Blutsbestandteile, z. B. der Thrombozyten. Es ist daher bevorzugt, dass die Vielzahl von Hohlfäden als Fadenbündel mit im wesentlichen parallelen Fäden vorliegen.

C5a ist ein Spaltprodukt des Plasmaproteins C5. Der Maximalwert der C5a Konzentration im Blut ist daher limitiert durch die Konzentration von C5 im Blutplasma, wobei die C5 Konzentration im Plasma starken individuellen Schwankungen unterliegt und etwa 40mg/l bis 150 mg/l betragen kann. Aufgrund des Molmassenverhältnisses von C5 zu C5a ergibt sich dadurch eine theoretische Maximalkonzentration von C5a im Blut von 9 mg/l.

Die Konzentration des Komplementaktivierungsproduktes C5a wird in Blutplasma ermittelt unter Verwendung eines Sandwich-ELISA (enzymgekoppelter Immunnachweis) der Firma DRG Diagnostics, Marburg. Nach Kontakt der Fäden mit humanem Spenderblut (5U/ml Heparin) wird zu verschiedenen Zeiten 1.8 ml Blut entnommen und mit 0.2 ml 100 mM EDTA-Lösung abgestoppt. Vor der Analyse wird entsprechend der Herstellervorschrift C5 ausgefällt (200µl Plasma + 200 µl Präzipitationsreagens). 50 µl Überstand wird in die Bestimmung eingesetzt. Die Nachweisempfindlichkeit des Assays liegt bei < 0.02 µg/l, die Wiederfindungsrate von C5a im Plasma bei 86-114% und der Variationskoeffizient bei 5-8% (Intraassay) bzw. 6-10% (Interassay). Die gemessene C5a Konzentration ist vom Blutvolumen und der Fadenoberfläche abhängig. Daher ist zur Ermittlung der C5a Konzentration bezogen auf die äußere Oberfläche der Fäden der absolute C5a Gehalt in der Probe zu bestimmen und in Relation zur äußeren Fadenoberfläche zu setzen. Dabei ist ein Verhältnis Blutvolumen (V) zu Fadenoberfläche (A) V/A von 0,3 L/m² einzuhalten. Die Ermittlung der flächenbezogenen C5a Konzentration erfolgt nach einer Behandlungsdauer von 3 h, d.h. die Blutprobe wird 3 h an der äußeren Oberfläche der Fäden entlang geführt, wobei eine lineare Strömungsgeschwindigkeit von 5 bis 30 cm/min einzuhalten ist. Da die Messergebisse spenderabhängig individuellen Schwankungen unterliegen, sollte die Stichprobenanzahl N mindestens 2 betragen und die Mittelwerte der Stichproben angegeben werden.

Ohne an die Theorie gebunden sein zu wollen, wird vermutet, dass bei der Behandlung entzündlicher Erkrankungen der Komplementaktivierung eine wichtige Bedeutung zukommt und dass eine Reduzierung der Leukozytenzahl in Kombination mit einer Komplementaktivierung wesentlich effektiver ist als eine Reduzierung der Leukozytenzahl allein. Dazu muss die Komplementaktivierung, bestimmt durch die Konzentration von C5a im Blut, oberhalb des erfindungsgemäßen Schwellenwertes liegen.

Ein Zusammenhang zwischen den Parametern Leukozytenzahl und C5a ist wahrscheinlich dadurch gegeben, dass bestimmte Leukozyten durch C5a aktiviert werden können. Die Aktivierung durch C5a und andere Faktoren bewirkt, dass die Zellen adhäsiver ("klebriger") werden und daher verstärkt an C5a erzeugende Oberflächen binden.

Es ist daher bevorzugt, dass die Hohlfäden eine weiter verstärkte Bildung des Komplementaktivierungsproduktes C5a in einer Konzentration von mindestens 75 µg pro m² Fadenoberfläche bewirken.

Besonders bevorzugt bewirken die Hohlfäden eine Bildung des Komplementaktivierungsproduktes C5a in einer Konzentration von mindestens 100 µg pro m² Fadenoberfläche.

Da die erforderliche C5a Bildung nicht nur abhängig ist vom Polymer, sondern auch von Beimischungen oder vom Substitutionsgrad des Polymers, umfasst die Bezeichnung "auf Basis organischer Polymere" die polymeren Werkstoffe als solche, Substitutionen, Mischungen davon, Copolymere dieser Werkstoffe sowie evtl. zugegebene Hilfsstoffe oder Additive, wie z.B. Hydrophilierungsmittel.

Bevorzugt weist die Anordnung der Hohlfäden eine spezifische Oberfläche zur Blutbehandlung zwischen 0,1 und 100 cm² Fadenoberfläche pro ml zu behandelnden Blutes, vorzugsweise zwischen 0,5 und 20 cm² Fadenoberfläche pro ml zu behandelnden Blutes auf. Die Menge des zu behandelnden Blutes ergibt sich aus der Dauer der Blutbehandlung und dem Volumenstrom.

Als Hohlfäden aus organischen Polymeren kommen solche aus natürlichen Polymeren oder aus Polymeren, die auf synthetischen Wege hergestellt wurden in Frage. Hohlfäden aus natürlichen Polymeren sind insbesondere solche auf Basis von zellulosischen Polymeren, was Hohlfäden, die sog. polymeranalogen Reaktionen unterzogen worden sind, ebenfalls umfasst. Beispiele für solche Hohlfäden auf Basis von Zellulose sind solche aus regenerierter Zellulose, Zelluloseazetat oder modifizierter Zellulose wie z.B. Zelluloseester, Zelluloseäther, mit Benzylgruppen modifizierte Zellulose (Benzylzellulose) oder mit Diethylaminoethyl modifizierte Zellulose oder Mischungen dieser zellulosischen Polymere. Mit Hohlfäden auf Basis von zellulosischen Polymeren wird im erfindungsgemäßen Verfahren eine hohe Reduzierung der Leukozytenzahl erreicht, eine besonders hohe Reduzierung wird mit Hohlfäden aus regenerierter Zellulose erhalten. Des Weiteren können auch Hohlfäden auf der Basis von Chitin, bzw. Chitosan zum Einsatz kommen.

Bei den organischen Polymeren handelt es sich auch um solche Polymere, die auf synthetischem Wege hergestellt werden. Für Hohlfäden aus synthetischen Polymeren können solche verwendet werden, die aus Polyolefinen, Polyamiden, Polyacrylnitril, Polycarbonaten oder Polyester sowie daraus gewonnen Modifikationen, Blends, Mischungen oder Copolymere dieser Polymere bestehen. Vorzugsweise werden solche verwendet, die auf Sulfonpolymeren, wie z.B. Polysulfon oder Polyethersulfon, basieren. Diesen Polymeren können weitere Polymere wie z.B. Polyethylenoxid, Polyhydroxyether, Polyethylenglykol, Polyvinylalkohol oder Polycaprolacton als Zusatzstoffe beigemischt werden. Die Hohlfäden können darüber hinaus noch eine Beschichtung mit einem Additiv aufweisen. Bevorzugt enthalten solche Hohlfäden ein Hydrophilierungsmittel, z.B. Polyvinylpyrrolidon oder auch hydrophile Modifikationen dieser Polymere.

Selbstverständlich eignet sich die erfindungsgemäße Vorrichtung nicht nur zur Reduzierung der Leukozytenzahl in Vollblut, sondern auch zur Reduzierung der Restleukozytenzahl in Blutplasma oder anderen Blutkonzentraten. Daher wird im Rahmen der vorliegenden Erfindung unter Blut Vollblut, Blutplasma oder ein Blutkonzentrat verstanden.

Es hat sich gezeigt, dass mit den angegebenen Fadenmaterialien in erster Linie Leukozyten reduziert werden. Insbesondere mit zellulosischen Fadenmaterialien wird hauptsächlich die Anzahl von Granulozyten und Monozyten reduziert. Lymphozyten werden mit zellulosischen Materialien nur geringfügig reduziert.

Es ist daher im Rahmen des erfindungsgemäßen Verfahrens möglich, gezielt bestimmte Zelltypen einer Klasse zu reduzieren, wie aus der Klasse der Leukozyten die Monocyten und Granulozyten und nicht die Lymphocyten. Weiterhin zeichnen sich Fadenmaterialien auf Basis von Zellulose dadurch aus, dass Thrombozyten nur in geringem Maße zurückgehalten werden.

Für bestimmte Anwendungsfälle könnte es vorteilhaft sein, zusätzlich Thrombozyten gezielt aus dem Blut zu entfernen. Für diese Anwendungsfälle eignen sich Fäden aus Polyethylenterephtalat (PET), Polysulfon oder Polyethersulfon. Sollte eine Reduzierung von Thrombozyten und Leukozyten gewünscht sein, bietet sich eine Kombination beispielsweise von Zellulosefäden und PET-Fäden für die erfindungsgemäße Vorrichtung an.

Die erfindungsgemäße Vorrichtung zur Entfernung von Leukozyten aus Blut wird anhand der folgenden Figuren näher erläutert. Die Figuren zeigen bevorzugte Ausführungsformen der erfindungsgemäßen Vorrichtung, die jedoch nicht einschränkend zu verstehen sind.

Es zeigen:
Figur 1a, 1b - Bevorzugte Ausführungsformen der erfindungsgemäßen Vorrichtung mit Ein- und Auslasseinrichtung am Gehäusemantel
Figur 2 - Bevorzugte Ausführungsform der erfindungsgemäßen Vorrichtung mit stirnseitiger Ein- bzw. Auslasseinreichtung

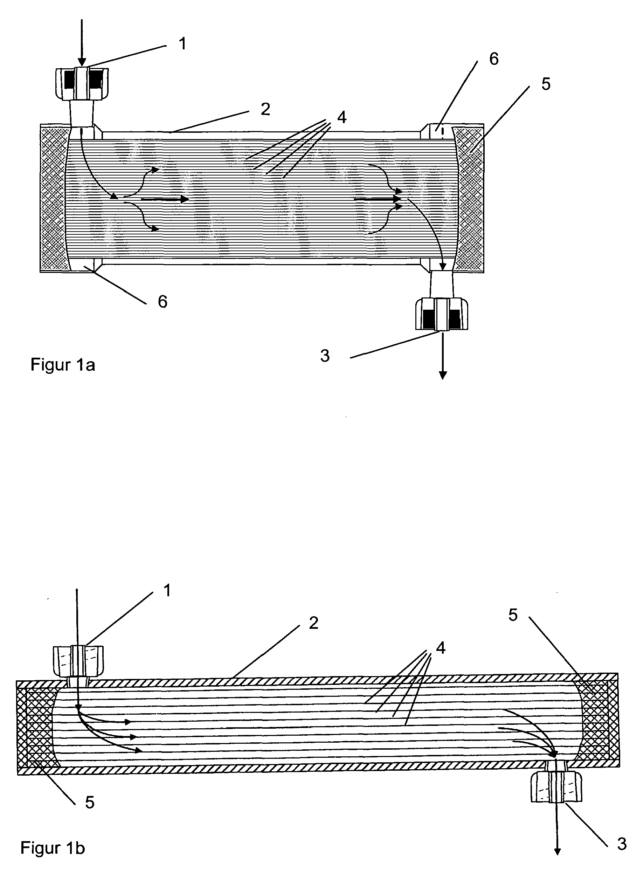

Figur 1a zeigt eine bevorzugte Ausführungsform der erfindungsgemäßen Vorrichtung mit Ein- und Auslasseinrichtung am Gehäusemantel. Die Einlasseinrichtung 1 befindet sich in dieser Ausführungsform an einem Ende des Gehäusemantels 2 und die Auslasseinrichtung 3 am anderen Ende des Gehäusemantels bei um 180° versetzter Anbringung. Für die Ein- bzw. Auslasseinrichtung können beispielsweise die gezeigten Luerlock Anschlüsse verwendet werden, es können aber auch alle anderen dem Fachmann bekannten Anschlüsse verwendet werden. Die Enden der Hohlfäden 4 sind in eine mit der Gehäuseinnenseite verbundenen Vergussmasse 5 eingebettet, so dass die Enden der Hohlfäden 4 in der Vergussmasse 5 liegen und durch die Vergussmasse 5 verschlossen sind. Die Lumina der eingebetteten Hohlfäden 4 sind nicht zugänglich, sondern nur die äußere Oberfläche der Hohlfäden. Die Hohlfäden 4 sind in der gezeigten bevorzugten Ausführungsform mit ihren Enden getrennt in Vergussmassen 5 eingebettet und erstrecken sich zwischen diesen Vergussmassen 5 im wesentlichen parallel. Im Bereich der Einlasseinrichtung 1, bzw. der Auslasseinrichtung 3 befinden sich Strömungsverteiler 6 in Form eines an dieser Stelle erweiterten Gehäusedurchmessers, die der besseren Blutverteilung dienen.

Figur 1b zeigt eine weitere bevorzugte Ausführungsform der erfindungsgemäßen Vorrichtung mit Einlasseinrichtung1 und Auslasseinrichtung 3 am Gehäusemantel und mit einem im Vergleich zur Figur 1a größerem Länge/Duchmesser Verhältnis von etwa 6:1.

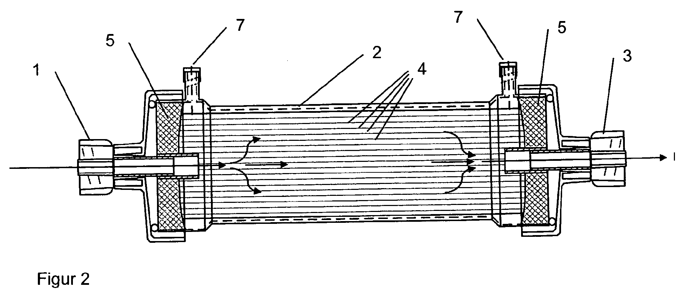

Figur 2 zeigt eine bevorzugte Ausführungsform der erfindungsgemäßen Vorrichtung mit Einlasseinrichtung 1 und Auslasseinrichtung 3, die mittig an den Stirnseiten des Gehäuses angebracht sind. Auch hier ist die Einlasseinrichtung 1 und Auslasseinrichtung 3 mit einem Luerlock Anschluss realisiert, es können aber auch alle anderen dem Fachmann bekannten Anschlüsse verwendet werden. Die Hohlfäden 4 sind auch in dieser bevorzugten Ausführungsform mit ihren Enden getrennt in Vergussmassen 5 eingebettet und erstrecken sich zwischen diesen Vergussmassen 5 im wesentlichen parallel. Aufgrund der stirnseitig angebrachten Ein- bzw. Auslasseinrichtung ist die Vielzahl der Hohlfäden 4 ringförmig mit ihren Enden um die Ein- bzw. Auslasseinrichtung herum in Vergussmasse eingebettet. Die in Figur 2 dargestellten Anschlüsse 7 am Gehäusemantel 2 sind verschlossen, könnten im Betrieb der Vorrichtung aber auch zur Entlüftung der Vorrichtung verwendet werden. Die Ein- bzw. Auslasseinrichtung ist so ausgebildet, dass sie zentral von der Stirnseite durch die Vergussmasse in das Gehäuseinnere, d.h. in den Außenraum um die Hohlfäden geführt wird. Zur besseren Verteilung des Blutes kann sowohl die Einlasseinrichtung 1 als auch die Auslasseinrichtung 3 im Gehäuseinneren als perforiertes Rohr ausgeführt sein, wobei dann das rohrförmige Ende der Ein- bzw. Auslasseinrichtung verschlossen ist, so dass einströmendes Fluid nur über den perforierten Mantel in den Außenraum um die Hohlfäden strömen kann.

## Patentansprüche

1. Vorrichtung zur Reduzierung von Leukozyten aus Blut umfassend eine Vielzahl von Hohlfäden (4) auf Basis organischer Polymere, wobei die Hohlfäden (4) der Vorrichtung ein Lumen und eine das Lumen umschließende Wand mit einer inneren, lumenseitigen Oberfläche und einer äußeren Oberfläche aufweisen, wobei die Hohlfäden der Vorrichtung in einem zylindrischen Gehäuse mit einer Einlasseinrichtung (1) und einer Auslasseinrichtung (3) vorliegen, wobei zwischen den Hohlfäden (4) und dem Gehäuse ein Außenraum gebildet wird, der über die Einlasseinrichtung (1) und die Auslasseinrichtung (3) für ein Fluid zugänglich ist und die Anordnung der Hohlfäden der Vorrichtung einen hohen Grad an Ordnung aufweist, wobei unter einem hohen Grad an Ordnung zu verstehen ist, dass ein hoher Anteil der Fäden entlang ihrer Erstreckungsrichtung nebeneinander angeordnet sind, **dadurch gekennzeichnet,**
**dass** die Enden der Hohlfäden der Vorrichtung durch Einbettung in eine Vergussmasse, durch Schweißen oder Kleben verschlossen sind, dass nur die äußere Oberfläche der Hohlfäden (4) der Vorrichtung für ein Fluid zugänglich und die Lumina der Hohlfäden (4) der Vorrichtung nicht für ein Fluid zugänglich sind und dass die Hohlfäden (4) der Vorrichtung auf Basis organischer Polymere eine Bildung des Komplementaktivierungsproduktes C5a in einer Konzentration von mindestens 10 µg pro m² äußerer Fadenoberfläche bewirken.

2. Vorrichtung zur Reduzierung von Leukozyten aus Blut nach Anspruch 1, **dadurch gekennzeichnet, dass** die Enden der Hohlfäden so in eine mit der Gehäuseinnenseite verbundenen Vergussmasse eingebettet sind, dass die Enden der Hohlfäden in der Vergussmasse liegen und durch die Vergussmasse verschlossen sind und dass die Hohlfäden mit ihren Enden getrennt in Vergussmassen eingebettet sind und sich zwischen diesen Vergussmassen im wesentlichen geradlinig erstrecken.

3. Vorrichtung zur Reduzierung von Leukozyten aus Blut nach einem oder mehreren der Ansprüche 1 oder 2, **dadurch gekennzeichnet, dass** sich die Einlasseinrichtung an einem Ende des Gehäusemantels und die Auslasseinrichtung am anderen Ende des Gehäuses befindet.

4. Vorrichtung zur Reduzierung von Leukozyten aus Blut nach einem oder mehreren der Ansprüche 1 oder 2, **dadurch gekennzeichnet, dass** die Ein- bzw. Auslasseinrichtung so ausgebildet ist, dass sie zentral von der Stirnseite durch die Vergussmasse in das Gehäuseinnere, d.h. in den Außenraum um die Hohlfäden geführt wird.

5. Vorrichtung zur Reduzierung von Leukozyten aus Blut nach einem oder mehreren der Ansprüche 1 bis 4, **dadurch gekennzeichnet, dass** der Außendurchmesser der Hohlfäden auf Basis organischer Polymere zwischen 150 µm und 2000 µm liegt.

6. Vorrichtung zur Reduzierung von Leukozyten aus Blut nach einem oder mehreren der Ansprüche 1 bis 5, **dadurch gekennzeichnet, dass** die Hohlfäden Hohlfäden mit dichter oder poröser Struktur sind und dass die Hohlfäden mit poröser Struktur eine maximale Porenweite von 0,1 µm aufweisen.

7. Vorrichtung zur Reduzierung von Leukozyten aus Blut nach einem oder mehreren der Ansprüche 1 bis 6, **dadurch gekennzeichnet, dass** der Füllgrad der Hohlfäden im Gehäuse im Bereich von 10% bis 70% liegt.

8. Vorrichtung zur Reduzierung von Leukozyten aus Blut nach einem oder mehreren der Ansprüche 1 bis 7, **dadurch gekennzeichnet, dass** die Hohlfäden auf Basis organischer Polymere in einer oder mehrerer Lagen vorliegen und dass die Fäden innerhalb einer Lage im wesentlichen parallel liegen.

9. Vorrichtung zur Reduzierung von Leukozyten aus Blut nach einem oder mehreren der Ansprüche 1 bis 8, **dadurch gekennzeichnet, dass** die Hohlfäden eine Bildung des Komplementaktivierungsproduktes C5a in einer Konzentration von mindestens 75 µg pro m² Fadenoberfläche Blut bewirken.

10. Vorrichtung zur Reduzierung von Leukozyten aus Blut nach einem oder mehreren der Ansprüche 1 bis 9, **dadurch gekennzeichnet, dass** die Hohlfäden auf Basis organischer Polymere aus regenerierter Zellulose, Zelluloseazetat oder mit Benzylgruppen modifizierter Zellulose (Benzylzellulose) bestehen.

11. Vorrichtung zur Reduzierung von Leukozyten aus Blut nach einem oder mehreren der Ansprüche 1 bis 9, **dadurch gekennzeichnet, dass** die Hohlfäden auf Basis organischer Polymere im Wesentlichen aus Polyethersulfon oder Polysulfon bestehen.

12. Vorrichtung zur Reduzierung von Leukozyten aus Blut nach einem oder mehreren der Ansprüche 1 bis 11, **dadurch gekennzeichnet, dass** die Anordnung einer Vielzahl von Hohlfäden zusätzlich auch Fäden aus Polyethylenterephthalat enthält.

## Claims

1. A device for reducing the number of leukocytes in blood comprising a plurality of hollow fibers (4) based on organic polymers, wherein the hollow fibers (4) of the device have a lumen and a wall surrounding the lumen, said wall having an internal surface facing the lumen and an external surface, wherein the hollow fibers of the device are arranged in a cylindrical housing with an inlet arrangement (1) and an outlet arrangement (3), wherein an outer space is formed between the hollow fibers (4) and the housing, which space is accessible for a fluid via the inlet arrangement (1) and the outlet arrangement (3), and the arrangement of the hollow fibers of the device shows a high degree of order, wherein a high degree of order is understood to mean that a high proportion of the fibers are arranged side by side along their direction of extension, **characterized in that**
the ends of the hollow fibers of the device are sealed by embedding in a sealing compound, or by welding or gluing, that only the external surface of the hollow fibers (4) of the device is accessible for a fluid and the lumina of the hollow fibers (4) of the device are not accessible for a fluid, and that the hollow fibers (4) of the device based on organic polymers cause a generation of the complement activation product C5a in a concentration of at least 10 µg per m² of external fiber surface.

2. A device for reducing the number of leukocytes in blood according to Claim 1, **characterized in that** the ends of the hollow fibers are embedded in a sealing compound connected to the inner side of the housing in such a way that the ends of the hollow fibers lie in the sealing compound and are sealed by the sealing compound, and that the hollow fibers are embedded with their ends separately in sealing compounds and extend essentially rectilinearly between these sealing compounds.

3. A device for reducing the number of leukocytes in blood according to one or more of Claims 1 or 2, **characterized in that** the inlet arrangement is located at one end of the housing shell and the outlet arrangement is located at the other end of the housing.

4. A device for reducing the number of leukocytes in blood according to one or more of Claims 1 or 2, **characterized in that** the inlet or outlet arrangement is designed in such a way that it is inserted centrally from the face through the sealing compound into the interior of the housing, i.e. in the outer space around the hollow fibers.

5. A device for reducing the number of leukocytes in blood according to one or more of Claims 1 to 4, **characterized in that** the outside diameter of the hollow fibers based on organic polymers lies between 150 µm and 2000 µm.

6. A device for reducing the number of leukocytes in blood according to one or more of Claims 1 to 5, **characterized in that** the hollow fibers are hollow fibers with impermeable or porous structure and that the hollow fibers with porous structure have a maximum pore size of 0.1 µm.

7. A device for reducing the number of leukocytes in blood according to one or more of Claims 1 to 6, **characterized in that** the fill ratio of the hollow fibers in the housing lies in the range of 10% to 70%.

8. A device for reducing the number of leukocytes in blood according to one or more of Claims 1 to 7, **characterized in that** the hollow fibers based on organic polymers are arranged in one or more layers and that the fibers within one layer lie essentially parallel.

9. A device for reducing the number of leukocytes in blood according to one or more of Claims 1 to 8, **characterized in that** the hollow fibers cause a generation of the complement activation product C5a in a concentration of at least 75 µg of blood per m² of fiber surface.

10. A device for reducing the number of leukocytes in blood according to one or more of Claims 1 to 9, **characterized in that** the hollow fibers based on organic polymers consist of regenerated cellulose, cellulose acetate, or cellulose modified with benzyl groups (benzyl cellulose).

11. A device for reducing the number of leukocytes in blood according to one or more of Claims 1 to 9, **characterized in that** the hollow fibers based on organic polymers consist essentially of polyether sulfone or polysulfone.

12. A device for reducing the number of leukocytes in blood according to one or more of Claims 1 to 11, **characterized in that** the arrangement of a plurality of hollow fibers also contains fibers made of polyethylene terephthalate.

## Revendications

1. Appareil permettant de faire baisser le nombre de leucocytes dans le sang, comprenant un grand nombre de fibres creuses (4) à base de polymère organique, lesquelles fibres creuses de l'appareil présentent une lumière et une paroi entourant la lumière et présentant une surface interne côté lumière et une surface externe, dans lequel appareil les fibres creuses sont placées dans une enveloppe cylindrique munie d'un dispositif d'entrée (1) et d'un dispositif de sortie (3), et entre les fibres creuses (4) et l'enveloppe est ménagé un espace extérieur aux fibres, accessible à un fluide par le dispositif d'entrée (1) et le dispositif de sortie (3), et les fibres sont disposées dans l'appareil avec un haut degré d'ordre, étant entendu qu'il faut comprendre, par ce « haut degré d'ordre », qu'une grande partie des fibres sont rangées les unes à côté des autres dans la direction dans laquelle elles s'étendent, **caractérisé en ce que** les extrémités des fibres creuses de l'appareil sont obturées par encastrement dans une masse de scellement, par soudure ou collage, **en ce que** seule la surface externe des fibres creuses (4) de l'appareil est accessible à un fluide, les lumières des fibres creuses (4) de l'appareil n'étant pas accessibles à un fluide, et **en ce que** les fibres creuses (4) à base de polymère organique de l'appareil provoquent la formation du produit d'activation du complément C5a en une concentration d'au moins 10 µg par mètre carré de surface externe des fibres.

2. Appareil permettant de faire baisser le nombre de leucocytes dans le sang, conforme à la revendication 1, **caractérisé en ce que** les extrémités des fibres creuses sont encastrées dans une masse de scellement attachée au côté interne de l'enveloppe de telle manière que les extrémités des fibres creuses se trouvent dans la masse de scellement et sont obturées par la masse de scellement, et **en ce que** les fibres creuses ont leurs extrémités encastrées séparément dans des masses de scellement et s'étendent pratiquement en ligne droite entre ces masses de scellement.

3. Appareil permettant de faire baisser le nombre de leucocytes dans le sang, conforme à l'une ou plusieurs des revendications 1 et 2, **caractérisé en ce que** le dispositif d'entrée se trouve à une extrémité de l'enveloppe et le dispositif de sortie à l'autre extrémité de l'enveloppe.

4. Appareil permettant de faire baisser le nombre de leucocytes dans le sang, conforme à l'une ou plusieurs des revendications 1 et 2, **caractérisé en ce que** le dispositif d'entrée, ou le dispositif de sortie, est agencé de telle manière qu'il mène depuis le côté face, au centre de la masse de scellement et à travers celle-ci, jusqu'à l'intérieur de l'enveloppe, c'est-à-dire dans l'espace extérieur aux fibres creuses et qui les entoure.

5. Appareil permettant de faire baisser le nombre de leucocytes dans le sang, conforme à l'une ou plusieurs des revendications 1 à 4, **caractérisé en ce que** le diamètre externe des fibres creuses à base de polymère organique vaut entre 150 µm et 2000 µm.

6. Appareil permettant de faire baisser le nombre de leucocytes dans le sang, conforme à l'une ou plusieurs des revendications 1 à 5, **caractérisé en ce que** les fibres creuses sont des fibres creuses dotées d'une structure compacte ou d'une structure poreuse, et **en ce que** les pores des fibres creuses de structure poreuse ont une taille d'au plus 0,1 µm.

7. Appareil permettant de faire baisser le nombre de leucocytes dans le sang, conforme à l'une ou plusieurs des revendications 1 à 6, **caractérisé en ce que** le taux de remplissage de l'enveloppe par les fibres creuses vaut de 10 % à 70 %.

8. Appareil permettant de faire baisser le nombre de leucocytes dans le sang, conforme à l'une ou plusieurs des revendications 1 à 7, **caractérisé en ce que** les fibres creuses à base de polymère organique se présentent en une ou plusieurs couche(s) et **en ce que** ces fibres, au sein d'une couche, sont pratiquement parallèles.

9. Appareil permettant de faire baisser le nombre de leucocytes dans le sang, conforme à l'une ou plusieurs des revendications 1 à 8, **caractérisé en ce que** les fibres creuses provoquent la formation du produit d'activation du complément C5a en une concentration d'au moins 75 µg par mètre carré de surface des fibres.

10. Appareil permettant de faire baisser le nombre de leucocytes dans le sang, conforme à l'une ou plusieurs des revendications 1 à 9, **caractérisé en ce que** les fibres creuses à base de polymère organique sont faites de cellulose régénérée, d'acétate de cellulose ou de benzyl-cellulose (cellulose modifiée avec des groupes benzyle).

11. Appareil permettant de faire baisser le nombre de leucocytes dans le sang, conforme à l'une ou plusieurs des revendications 1 à 9, **caractérisé en ce que** les fibres creuses sont faites essentiellement de poly(éther sulfone) ou de polysulfone.

12. Appareil permettant de faire baisser le nombre de leucocytes dans le sang, conforme à l'une ou plusieurs des revendications 1 à 11, **caractérisé en ce que** l'arrangement d'un grand nombre de fibres creuses comporte en outre des fibres en poly(téréphtalate d'éthylène).
